# EUROPEAN PATENT SPECIFICATION

(11) **EP 1 722 689 B1**
(45) Date of publication and mention of the grant of the patent: **29.07.2009**
(21) Application number: 05722993.2
(22) Date of filing: 09.02.2005
(51) Int. Cl.: A61B 17/00, A61B 17/28

(54) **LIGHTED DISSECTOR**
BELEUCHTETER DISSEKTOR
INSTRUMENT DE DISSECTION LUMINEUX

(30) Priority: 09.03.2004 US 796901
(43) Date of publication of application: 22.11.2006
(73) Proprietor: AtriCure Inc., West Chester, OH 45069 (US)
(72) Inventor: PALMER, Joetta, Renee, Mason, OH 45040 (US); WOLF, Randall, Kevin, Cincinnati, OH 45243 (US); SCHNEEBERGER, Eric, William, Cincinnati, OH 45208 (US); ALEXANDER, Patrick, Jerome, Cincinnati, OH 45239 (US); DIVELBISS, Daniel, William, Fredericktown, OH 43019 (US); WINKLER, Matthew, Joseph, Liberty Township, OH 45011 (US); HARP, Adam, Ray, Cincinnati, OH 45245 (US); NUCHOLS, Richard, Paul, Loveland, OH 45140 (US)
(74) Representative: Samson & Partner
(86) International application number: PCT/US2005/004492
(87) International publication number: WO 2005/092201

(56) References cited:
- US-A- 5 522 788
- US-A- 5 571 119
- US-A- 5 649 957
- US-A- 5 797 959
- US-B1- 6 203 557

## Description

### BACKGROUND

The present invention relates to surgical tools, and more specifically to surgical dissectors. In the broadest sense, dissectors are used to cut apart or separate tissue. For instance, during an operation dissectors can be used to separate different structures along natural lines by dividing the connective tissue framework. Dissectors can take a wide variety of shapes and sizes. For example, some dissecting surfaces are blunt (e.g., rounded, fanned, or the like) while other dissectors have sharpened surfaces (e.g., needles, lances, blades, and the like). No one, however, has previously made or used dissector in accordance with the present invention.

US 5,522,788 discloses a blunt dissector with a flexible distal end portion including a finger-like distal element having a recess in the upper surface thereof. The dissector includes a cannula or channel element terminating in the recessed portion of the distal element to permit the insertion of various instruments or a light guide or fiber optic element for illuminating the surgical site.

### SUMMARY OF THE INVENTION

According to an aspect of the present invention, a surgical dissector in accordance to the subject-matter of independent claim 1 is provided. Further aspects of the invention are set forth in the dependent claims, the following description and the drawings.

According to an embodiment of the invention, a surgical dissector comprising an elongate shaft having a proximal end and a distal end. A blunt dissection tip is positioned on the distal end of the elongate shaft. A light source emits a visible energy, such as a diffuse and/or unfocused white light, from the blunt tip. The shaft may take a variety of shapes, including being rigid, flexible, malleable, straight, bent, curved, articulated, and/or segmented. In addition, the shaft may include one or more functional components.

The foregoing brief description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

### BRIEF DESCRIPTION OF DRAWINGS

While the specification concludes with claims which particularly point out and distinctly claim the invention, it is believed the present invention will be better understood from the following description taken in conjunction with the accompanying drawings, in which like reference numerals identify the same elements and in which:
Fig. 1 illustrates an example of a dissector;
Fig. 2 illustrates another example of a dissector; and
Fig. 3 illustrates a partial cross-sectional view of a portion of the dissector shown in Fig. 2.

### DETAILED DESCRIPTION

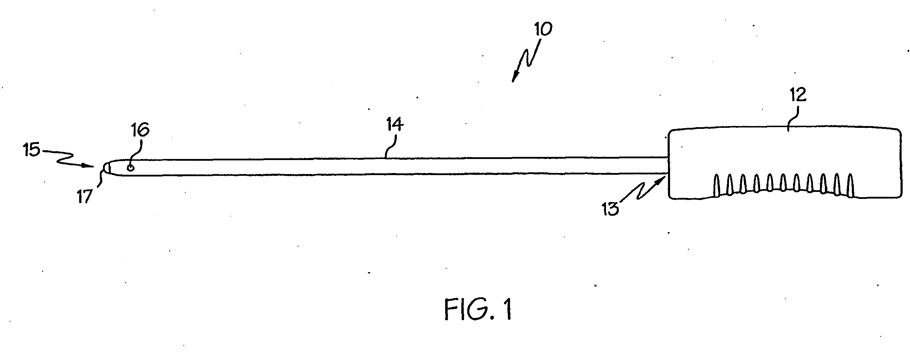

Fig. 1 illustrates an example of a dissector 10. The dissector 10 includes and elongate shaft 14 having a proximal end 13 and a distal end 15. A handle 12 is connected to the shaft 14 at the proximal end 13. In the present example, the shaft 14 is made from stainless steel, but numerous other materials known in the art may also be employed. The shaft 14 and has a circular cross section along its length and the distal end 15 is a blunt and rounded tip, which tip may be smooth or rough. Any portion of the shaft 14 can be used for dissecting tissue. It should be appreciated, however, that variable cross-sectional shapes are also contemplated, such as a fanned or flatted portions. In addition, the distal end 14 could have numerous other geometries, such as a Y-shaped tip.

As shown in this example, the shaft is substantially straight; however, the shaft 14 can take a variety of alternative shapes. For instance, the shaft 14 could be bent, curved, arced, undulated, helical, twisted, and the like. Further, the shaft 14 could be moveable, such as having one or more articulated joints or multiple segments. In addition, the shaft 14 could be rigid, flexible or malleable, either along its entire length or only along a portion. The shaft 14 includes an optional hole 16 so that sutures or other devices may be attached. In an alternative embodiment, the distal end 15 includes a step or barb onto which an elastomeric tube could be connected. The shaft 14 may also include one or more functional components to facilitate dissection, such as a grasper, an inflatable balloon, an expanding cage or arm, retractors, an ultrasonic emitter, a retractable sharped surface, an endoscope, a port for water jet dissection, a guide wire, a oxygen content sensor, a working lumen, a fixed or rotating knurled ball, or other components known in the art. The functional components can be integral to the dissector 10 or could be separable, such as removable or interchangeable tips.

A light source 17 is positioned at the distal end 15 of the shaft 14. The light source 17 emits a visible energy. In the present example the visible energy is a diffuse and substantially unfocused. The wavelength of the visible energy may vary, including for instance being substantially white, green, red, or other color. The light source 17 in this example takes the form of an light emitting diode (LED) positioned on the distal tip of the shaft 14. Alternative lights sources may also be used, including without limitation incandescent, fluorescent, laser, infrared and the like. The visible energy can originate directly from the light source 17 or can originate from a position remote to the distal end 15 (e.g., in the shaft 14, handle 12, or external to the dissector). For instance, the light can be delivered to the distal end 15 via fiber optics or a light pipe. While the light source 17 in the present example emits light from a point positioned on or near the distal end 15, it is also contemplated that light could be emitted from multiple points or from an area, such as along a segment of the shaft 14.

The visible energy has sufficient luminous intensity to pass through tissue. Suitable luminous intensity will vary depending upon the tissue being dissected. Some exemplary ranges of luminous intensity include between about 20 lux and about 50,000 lux, 300 lux and about 1500 lux, between about 500 lux and about 1500 lux, and between about 700 lux and about 1300 lux. Note that these ranges are merely illustrative and not limiting. The light source 17 here is powered by a battery positioned in the handle 12, but it could be powered using different configurations such as a remote tethered power source.

One illustrative use of the dissector 10 is to separate two adjacent tissues. The distal end 15 is position at the junction of the two tissues. As the shaft is moved between the tissues, the two tissue separate and become dissected. By laterally moving the shaft, a wider dissection can be achieved. In many cases, one or both of the tissues being dissected may obstruct the surgeon's line of sight, such that they cannot visually identify the location of the distal end 15. In such situations, the locating the distal end 15 can be located by observing the diffuse visible energy passing through the obstructing tissue. Accordingly, the operator will have better control and accuracy while dissecting. In addition, by observing the visible energy passing through the tissue the surgeon can differentiate between different tissues. The light source 17 can continuously emit, periodically emit (e.g., a slow or rapid sequence such as with a strobe), or selectively emit the visible energy (e.g., activate the light source only when desired). Being able to locate the distal end 15 which would otherwise be visually obstructed and/or being able to differentiate tissue is particularly useful when dissecting fragile tissue or near sensitive organs.

In addition to transillumination of tissue, the visible energy can be used to directly illuminate a surgical area. For instance, a surgeon may desire to illuminate a surgical field, In one variation, the shaft 14 has a lumen and the distal end 15 is transparent. In such embodiment, an endoscope can be threaded through the lumen and the surgeon may visualize a patient's anatomy from the perspective of the distal end 15 while being illuminated by the light source 17.

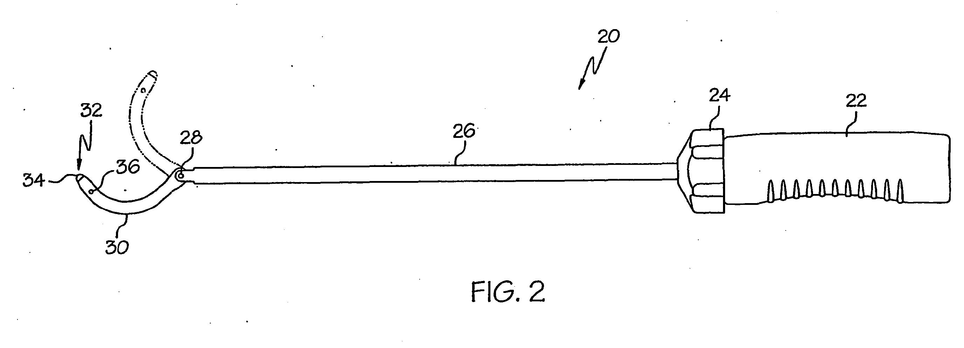

Fig. 2 illustrates another example of a dissector 20. The dissector 20 comprises an elongate shaft 26 with a handle 22 connected to the proximal end of the shaft 26. The shaft 26 is articulated and includes an arcuate and elongate segment 30 distal the joint 28 and a substantially straight segment proximal the joint 28. The segment 30 has blunt and rounded distal end 32, and includes an optional suture hole 36. The segment 30 pivots about a joint 28. In the present example, the segment 30 pivots about a single axis of rotation, but more complicated joints may also be employed. A knob 24 is positioned on handle 22 that actuates and controls the position of the segment 30 by manually rotating the knob 24. The present figure illustrates two exemplary angular positions. The segment 30 shown in solid is positioned in a "straight" or "back" position where the distal end 32 is substantially aligned with aligned with axis of the shaft 26 (i.e, at 0°). As shown in phantom, the segment 30 is in a "bent" or "forward" position where the distal end 32 is positioned at about 75° from the axis of the shaft 26. The segment 30 can pivot to any position between the extremes of 0°-75°. Alternatively, the segment 30 can be pivoted outside that range (i.e., less than 0° and/or greater than 75°). For instance, one embodiment pivots between -30° and 140°.

A light source 34 emits visible energy from the distal end 32 of the segment 30. The light source 34 in this example emits a substantially unfocused and diffuse light. While a variety of different light sources 34 may be employed, the present embodiment uses a model NSPW500BS white LED produced by NICHIA positioned on the distal end 32. A battery in the handle 22 powers the light source 34.

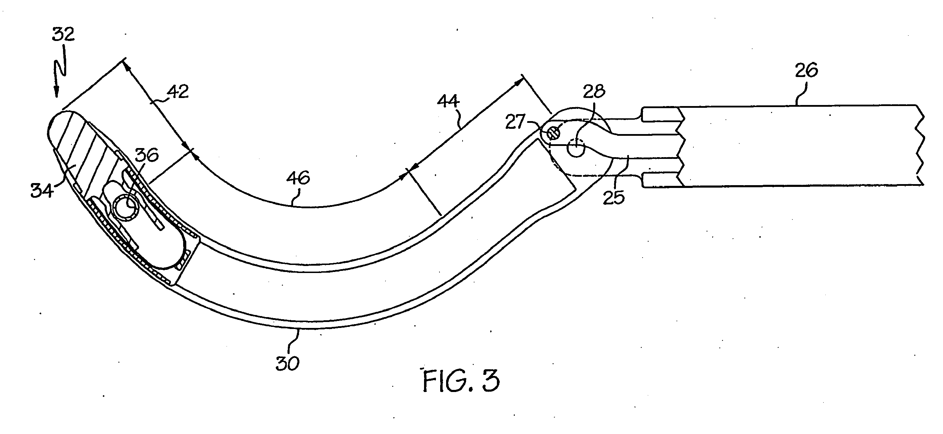

Fig. 3 illustrates a partial cross-sectional view of the dissector 20. The light source 34 is partially encased within the segment 30 wall and is exposed to define the blunt tip geometry of the distal end 32. A connection rod 25 is positioned in the shaft 26 and connects to the proximal end of the segment 30 with a pin 27 offset from the axis of rotation of the joint 28. The other end of the rod (not shown) is connected to a worm screw that engages a threaded nut connected to the knob 24. Accordingly, the operator can manually rotate the knob 24 which axially moves the rod 25, which in turn pivots the segment 30. One advantage of this embodiment is that the after the surgeon releases the knob 24, the angular position of the segment 30 relative the shaft 26 remains secure and relatively rigid. While the present actuation arrangement has certain advantages, other actuation arrangements known in the art may also be used, including without limitation scissors-type handles, rolling wheels, slide levers, spring mechanisms.

While the geometry of the arcuate segment 30 may vary significantly based on the targeted anatomy, the following describes the geometry of present example. The segment 30 in the present example has a smooth outer surface and a substantially circular cross-sectional shape that tapers slightly toward the distal end 32. The nominal diameter is about 3/16 inch, but a variety of other diameters may be used, including without limitation diameters ranging from 0.5 to 0.075 inches. The length of the segment 30 measured from the distal end 32 to the joint 28 ranges from about 2 to 2.5 inches, but the length may be extended outside this range depending upon the intended medical procedure. For instance, the length may also be between about 0.5 to 4 inches. The arcuate shape of the segment 30 in this example includes an arc portion 46, a proximal linear portion 44, and a distal linear portion 42. The radius of the arc portion 46 shown here is about 1 inch and swept about 90°; however, other arc geometries may be used, including without limitation arc radii ranging from 0.25 to 3 inches and swept 30° to 180°. The proximal linear portion 44 here is about 0.5 inches long and the distal linear portion is about 0.25 inches long. The dimensional range of the linear portions 42, 44 may also be varied substantially. Naturally, the foregoing geometries are merely illustrative and should not be considered limiting.

The dissector 20 of the present example is well-suited for separating and/or isolating a variety of tissues, during both open and/or minimally invasive procedures. Some exemplary procedures include, without limitation:
- Isolate pulmonary arteries and branches;
- Isolate pulmonary veins and branches;
- During billiary surgery with gall bladder, separating the vein from artery and/or separating the bile duct from the vascular pedicle;
- Isolate aorta, such as for retroperiteneal isolation of thoracic or abdominal aorta;
- Isolate renal pedicle;
- Isolate illiac vessel;
- Isolate femoral artery from vein;
- Isolate arch vessels;
- Isolate carotids;
- Isolate rectum from pelvic floor through peritoneum; and
- Isolate other tubular structures from connective tissue.

The following describes an exemplary procedure using the dissector 20 to separate the left or right pair of pulmonary veins adjacent the left atrium. The procedure may be performed during open or minimally invasive surgery. With the segment 30 in a substantially straight position, the distal end 32 of the segment 30 is positioned adjacent the junction of one of the pulmonary veins (superior or inferior) and the left atrium. The distal end 32 is advanced around the posterior of the pair of pulmonary veins while simultaneously changing the angular position of the segment 30 in the forward direction. The distal end 32 continues to advance until it emerges beyond the other adjacent pulmonary vein (the inferior or superior, as the case may be). The advancement of the distal end separates the pair of pulmonary veins from the pericardial reflections, thus creating a path between the pulmonary veins and the pericardium. The path can be widened by gently rotating back and forth the handle 22 while the segment 30 is in an articulated position, which will sweep the segment 30 and further separating the tissue and widen the path.

If the light source 34 is used, It has several useful benefits during the procedure. One benefit is to illuminate the surgical area during the initial approach and positioning of the distal end 32. Another benefit is to locate the distal end 32 during the procedure. While advancing, the distal end 32 is often obstructed from sight by the surrounding tissue. The light emitting from the light source 34 passes through the obstructing tissue and the surgeon can visually locate the distal end 32 by observing such light. Still another benefit of the light source 34 Is to differentiate between the various tissue. By observing light passing through tissue, the surgeon can discern if the distal end is approaching or contacting targeted or untargeted tissue. Accordingly, the surgeon has greater control and accuracy while dissecting the area.

One reason to dissect the pulmonary veins is as part of a procedure to treat atrial fibrillation. After the distal end 32 emerges beyond both pulmonary veins, further advancement and articulation will expose the distal end 32. A guide is then attached to the segment 30. For example, the guide may take the form of a suture or umbilical tape threaded through the hole 36. In another example, the guide may be a flexible catheter (such as a BARDIA urethral catheter) fitted over the distal end 32. The segment 30 is then reversed back through the path while pivoting the segment 30 in the backward direction, thus threading the guide through the path resulting in a sling around the pulmonary veins. The guide is then attached to one jaw of a clamping ablation device (including without limitation the devices disclosed in U.S. Patent 6,517,536). By pulling the other end of the guide, the jaw can be accurately positioned in the path and the pulmonary veins are interposed between the ablation jaws. The jaws can then be closed and the targeted tissue ablated.

Having shown and described various embodiments of the present invention, further adaptations of the systems described herein can be accomplished by appropriate modifications by one of ordinary skill in the art without departing from the scope of the present invention. Several of such potential modifications have been mentioned, and others will be apparent to those skilled in the art. Accordingly, the scope of the present invention should be considered In terms of the following claims and is understood not to be limited to the details of structure and operation shown and described in the specification and drawings.

## Claims

1. A surgical dissector (20) for dissecting a tissue at a selected site, comprising:
a) an elongated shaft (26) comprising a proximal end portion and a distal end portion (30);
b) the shaft including a tissue dissecting surface carried at the distal end portion of the shaft and including a distal end (32);
c) the shaft being articulable at a location proximal to the dissecting surface, and the dissector including an actuator (24) operable to remotely position the dissecting surface relative to a more proximal portion of the shaft; and
d) a light emitter (34) located in the distal end of the shaft and disposed to emit light through the dissection surface.

2. The surgical dissector of claim 1, wherein the shaft includes one or more articulation joints (28) carried by the shaft to permit the distal end portion to move relative to the more proximal portion of the shaft.

3. The surgical dissector of claim 2, wherein the actuator maintains an orientation at any given position within an actuation range.

4. The surgical dissector of claim 2, wherein the position of the dissecting surface relative to the shaft is communicated by the actuator.

5. The surgical dissector of claim 1, wherein the dissecting surface comprises a blunt distal end of the distal end portion.

6. The surgical dissector of claim 5, in which the blunt dissector tip has a generally circular cross section.

7. The surgical dissector of claim 1, wherein the distal end portion includes an arc portion (46).

8. The surgical dissector of claim 1, wherein the distal end portion is between about 2 inches (about 5.08cm) and 2.5 inches (about 6.35cm) in length.

9. The surgical dissector of claim 1, wherein the shaft comprises a straight portion proximal to the articulation joint.

10. The surgical dissector of claim 9, wherein the dissecting surface includes a blunt distal end of the distal end portion and the distal end portion is adapted to pivot between a first position where the dissecting surface is substantially aligned with axis of the straight portion, and a second position where the dissecting surface is at an angle relative the axis of the straight portion.

11. The surgical dissector of claim 1, in which the visible energy for the light emitter originates from the light source.

12. The surgical dissector of claim 1 in which the visible energy for the light source originates from a position remote to the distal end of the shaft.

13. The surgical dissector of claim 1, in which the light source for the light emitter is generated externally and communicated to the distal end portion.

14. The surgical dissector of claim 1, in which the light emits from multiple points.

15. The surgical dissector of claim 1, wherein the luminous intensity is greater than about 300 lux.

## Patentansprüche

1. Chirurgischer Dissektor (20) zum Durchtrennen eines Gewebes an einer ausgewählten Stelle, umfassend:
a) eine längliche Stange (26), die einen proximalen Endbereich und einen distalen Endbereich (30) umfasst;
b) wobei die Stange eine Gewebetrennfläche umfasst, die am distalen Endbereich der Stange getragen wird und ein distales Ende (32) umfasst;
c) wobei die Stange an einer Stelle proximal zu der Trennfläche gelenkig ist und der Dissektor einen Aktuator (24) umfasst, der bedienbar ist, um aus der Entfernung die Trennfläche bezüglich eines proximaleren Bereichs der Stange zu positionieren; und
d) einen Lichtemitter (34), der im distalen Ende der Stange angeordnet ist und ausgestaltet ist, Licht durch die Trennfläche zu emittieren.

2. Chirurgischer Dissektor nach Anspruch 1, bei welchem die Stange eine oder mehr Gelenkverbindungen (28) umfasst, die von der Stange getragen werden, um zu ermöglichen, dass der distale Endbereich bezüglich des proximaleren Bereichs der Stange beweglich ist.

3. Chirurgischer Dissektor nach Anspruch 2, bei welchem der Aktuator eine Orientierung an jeder Position in einem Betätigungsbereich beibehält.

4. Chirurgischer Dissektor nach Anspruch 2, bei welchem die Position der Trennfläche bezüglich der Stange durch den Aktuator übertragen wird.

5. Chirurgischer Dissektor nach Anspruch 1, bei welchem die Trennfläche ein stumpfes distales Ende des distalen Endbereichs umfasst.

6. Chirurgischer Dissektor nach Anspruch 5, bei welcher die stumpfe Dissektorspitze einen allgemein runden Querschnitt aufweist.

7. Chirurgischer Dissektor nach Anspruch 1, bei welchem der distale Endbereich einen bogenförmigen Bereich (46) umfasst.

8. Chirurgischer Dissektor nach Anspruch 1, bei welchem der distale Endbereich zwischen ungefähr 2 Zoll (ungefähr 5,08 cm) und 2,5 Zoll (ungefähr 6,35 cm) lang ist.

9. Chirurgischer Dissektor nach Anspruch 1, bei welchem die Stange einen geraden Bereich proximal zur Gelenkverbindung umfasst.

10. Chirurgischer Dissektor nach Anspruch 9, bei welchem die Trennfläche ein stumpfes distales Ende des distalen Endbereichs umfasst und der distale Endbereich ausgestaltet ist, zwischen einer ersten Position, bei welcher die Trennfläche im Wesentlichen fluchtend mit der Achse des geraden Bereichs angeordnet ist, und einer zweiten Position, bei welcher die Trennfläche in einem Winkel bezüglich der Achse des geraden Bereichs steht, zu schwenken.

11. Chirurgischer Dissektor nach Anspruch 1, bei welchem die sichtbare Energie für den Lichtemitter von der Lichtquelle ausgeht.

12. Chirurgischer Dissektor nach Anspruch 1, bei welchem die sichtbare Energie für die Lichtquelle von einer Position ausgeht, die von dem distalen Ende der Stange entfernt ist.

13. Chirurgischer Dissektor nach Anspruch 1, bei welchem die Lichtquelle für den Lichtemitter außerhalb erzeugt wird und zu dem distalen Endbereich übertragen wird.

14. Chirurgischer Dissektor nach Anspruch 1, bei welchem das Licht von mehreren Punkten ausstrahlt.

15. Chirurgischer Dissektor nach Anspruch 1, bei welchem die Lichtintensität mehr als ungefähr 300 Lux beträgt.

## Revendications

1. Instrument de dissection chirurgical (20) pour disséquer un tissu sur un site choisi, comprenant :
a) une tige allongée (26) comprenant une partie d'extrémité proximale et une partie d'extrémité distale (30) ;
b) la tige comprenant une surface de dissection d'un tissu portée à la partie d'extrémité distale de la tige et comprenant une extrémité distale (32) ;
c) la tige pouvant être articulée sur un site proximal par rapport à la surface de dissection et l'instrument de dissection comprenant un actionneur (24) pouvant être utilisé de façon à placer à distance la surface de dissection par rapport à une partie plus proximale de la tige ; et
d) un émetteur de lumière (34) situé à l'extrémité distale de la tige et conçu pour émettre de la lumière sur la surface de dissection.

2. Instrument de dissection chirurgical selon la revendication 1, dans lequel la tige comprend une ou plusieurs articulations (28) réalisées par la tige pour permettre à la partie d'extrémité distale d'être mobile par rapport à la partie plus proximale de la tige.

3. Instrument de dissection chirurgical selon la revendication 2, dans lequel l'actionneur conserve une orientation sur une position donnée quelconque dans un intervalle d'actionnement.

4. Instrument de dissection chirurgical selon la revendication 2, dans lequel la position de la surface de dissection par rapport à la tige est communiquée par l'actionneur.

5. Instrument de dissection chirurgical selon la revendication 1, dans lequel la surface de dissection comprend une extrémité distale émoussée de la partie d'extrémité distale.

6. Instrument de dissection chirurgical selon la revendication 5, dans lequel la pointe de l'instrument de dissection émoussé a une section croisée généralement circulaire.

7. Instrument de dissection chirurgical selon la revendication 1, dans lequel la partie d'extrémité distale comprend une partie arquée (46).

8. Instrument de dissection chirurgical selon la revendication 1, dans lequel la partie d'extrémité distale a une longueur entre environ 2 pouces (environ 5,08 cm) et 2,5 pouces (environ 6,35 cm).

9. Instrument de dissection chirurgical selon la revendication 1, dans lequel la tige comprend une partie linéaire proximale par rapport à l'articulation.

10. Instrument de dissection chirurgical selon la revendication 9, dans lequel la surface de dissection comprend une extrémité distale émoussée de la partie d'extrémité distale et la partie d'extrémité distale est adaptée pour pivoter entre une première position dans laquelle la surface de dissection est substantiellement alignée avec l'axe de la partie linéaire, et une seconde position dans laquelle la surface de dissection forme un angle par rapport à l'axe de la partie linéaire.

11. Instrument de dissection chirurgical selon la revendication 1, dans lequel l'énergie visible de l'émetteur de lumière provient de la source de lumière.

12. Instrument de dissection chirurgical selon la revendication 1, dans lequel l'énergie visible de la source de lumière provient d'une position distante par rapport à l'extrémité distale de la tige.

13. Instrument de dissection chirurgical selon la revendication 1, dans lequel la source de lumière de l'émetteur de lumière est générée au niveau externe et est communiquée à la partie d'extrémité distale.

14. Instrument de dissection chirurgical selon la revendication 1, dans lequel la lumière est émise à partir de points multiples.

15. Instrument de dissection chirurgical selon la revendication 1, dans lequel l'intensité lumineuse est supérieure à environ 300 lux.
